# EUROPEAN PATENT APPLICATION

(11) **EP 4 582 142 A1**
(43) Date of publication of application: **09.07.2025**
(21) Application number: 23859817.1
(22) Date of filing: 27.06.2023
(51) Int. Cl.: A61P 17/16, A61Q 19/00, A61Q 19/08, A61K 9/133, A61K 47/24, A61K 8/02, A61K 8/55, A61K 31/66

(54) **PORE IMPROVER AND SKIN IMPROVER**

(30) Priority: 01.09.2022 JP 2022138998; 30.11.2022 JP 2022190929
(71) Applicant: T. Hasegawa Co., Ltd., Tokyo 103-8431 (JP)
(72) Inventor: OCHI, Takao, Tokyo 103-8431 (JP)
(74) Representative: Zacco GmbH
(86) International application number: PCT/JP2023/023806
(87) International publication number: WO 2024/048047

(57) **Abstract**

Provided is a pore improving agent comprising a composition containing a lipid membrane structure formed from a phospholipid having an acid value of 5 mg KOH/g or more. Also provided is a skin improving agent comprising a composition containing a lipid membrane structure formed from a phospholipid having an acid value of 5 mg KOH/g or more.

## Description

### Technical Field

The present invention relates to a pore improving agent and a skin improving agent having a formed lipid membrane structure containing a specific phospholipid.

### Background Art

Pore-related skin concerns such as visible pores and acnes rank high in various consumer surveys every year. Examples of conventional care for visible pores include physical removal of keratin plugs using sheet packs or instruments, tightening with astringents, and dissolution or removal of keratin plugs using cleansing items containing solvents, surfactants, or scrubbing agents. These products, albeit placing a heavy burden on the skin, are either insufficiently effective or have only temporary effects, and more care with the products accumulates a heavier burden on the skin, leading to further skin problems. As for acnes, the number of acnes caused by sudden change in skin environments or physical stimulation, called skin irritation due to mask, has been increasing in recent years. Acnes are difficult to prevent, and the only way to deal with developed acnes is medical treatment or medication, which however leads to further skin problems such as scarring or adverse reactions of the medication responsible for dryness or rough skin.

As previous pore improving agents that improve visible pores or acnes, for example, Patent Literature 1 discloses a parakeratosis suppressing agent, a pore tightening agent, or a rough skin preventing or improving agent consisting of one or two or more compounds selected from the group consisting of a specific β-alanine derivative and a salt thereof. Patent Literature 2 reports that visible pores can be effectively improved by using a specific plant extract in combination with retinol-based components such as retinol and a retinol derivative.

Meanwhile, Patent Literature 3 reports that a lipid membrane structure composed mainly of a phospholipid is useful for skin care as a carrier for active ingredients, though it is not known that the lipid membrane structure is useful as a pore improving agent.

The number of consumers who are aware of sensitive skin is on the rise, and self-care items targeting sensitive skin have also been generalized in recent years. Previous care for sensitive skin has often been preoccupied with blending of an anti-inflammatory agent such as dipotassium glycyrrhizinate and superficial protective care that minimizes skin irritation, and has made insufficient approaches to not only concerns about sensitive skin, such as skin redness, itching, or rough skin, but various other concerns (wrinkles, dullness, acnes, sagging, and dark circle), etc. Even among consumers who have previously been rarely aware of sensitive skin, the number of skin problems caused by sudden change in skin environments or physical stimulation, called skin irritation due to mask, has been on the rise in recent years. Meanwhile, a liposome, a lipid membrane structure composed mainly of a phospholipid, is known to be useful for skin care as a carrier for active ingredients (Patent Literature 3).

### Citation List

### Patent Literature

Patent Literature 1: Japanese Patent Laid-Open No. 2012-21026
Patent Literature 2: Japanese Patent Laid-Open No. 2014-227374
Patent Literature 3: Japanese Patent No. 6778306

### Summary of Invention

### Technical Problem

The pore tightening agent, etc. described in Patent Literature 1 is insufficiently effective as an active ingredient alone and is therefore used in combination with high-concentration ethanol upon application to continuous use tests for humans, leaving problems such as unsafe use for humans with sensitive skin or skin problems and a long time for the agent to exert its effects.

The composition for pore improvement described in Patent Literature 2 employs a plant extract obtained by extraction with high-concentration ethanol in combination with retinol or its derivative, which largely influences the skin, for example, leaving problems such as unsafe use for humans with sensitive skin or skin problems and a long time for the composition to exert its effects.

Furthermore, as described above, although a lipid membrane structure composed mainly of a phospholipid is known to be useful for skin care as a carrier for active ingredients, its effects as a skin improving agent, including a rough skin preventing effect and a skin redness, dullness, or wrinkle improving effect are not known.

In light of these circumstances, an object of the present invention is to provide a pore improving agent that prevents any one or more of problems related to visible pores such as pore blackhead, large pores or sagging pores, and clogging by keratin plugs, and problems related to pore inflammation such as acnes, and also improves conditions of the problems.

Another object of the present invention is to provide a skin improving agent that safely prevents rough skin and improves or prevents conditions of skin problems such as skin redness, dullness, and wrinkles, with less burden even on sensitive skin without requiring special treatment or drugs.

### Solution to Problem

The present inventors have conducted diligent studies and consequently completed the present invention by finding that the objects can be attained by using a lipid membrane structure formed from a phospholipid having a specific acid value as a pore improving agent or a skin improving agent.

Specifically, the present invention is as follows.
[1] A pore improving agent comprising a composition containing a lipid membrane structure formed from a phospholipid having an acid value of 5 mg KOH/g or more.
[2] The pore improving agent according to [1], wherein the phospholipid is a hydrogenated phospholipid.
[3] The pore improving agent according to [1] or [2], wherein the lipid membrane structure is a single-layer lamellar structure.
[4] The pore improving agent according to [1] or [2], wherein an average hydrodynamic diameter of the lipid membrane structure is 200 nm or smaller.
[5] The pore improving agent according to [1] or [2], wherein the pore improving agent has a pore tightening effect.
[6] The pore improving agent according to [1] or [2], wherein the pore improving agent has a sebum secretion regulating effect.
[7] The pore improving agent according to [1] or [2], wherein the pore improving agent has an effect of surfacing or shedding keratin plugs or an effect of suppressing keratin plug formation, or both.
[8] The pore improving agent according to [1] or [2], wherein the pore improving agent has an effect of soothing acnes or an effect of suppressing acne development, or both.
[9] The pore improving agent according to [1] or [2], wherein the pore improving agent has a pore blackhead improving effect.
[10] The pore improving agent according to [1] or [2], wherein the pore improving agent has an effect of improving makeup adherence or an effect of improving makeup durability, or both.
[11] A skin improving agent comprising a composition containing a lipid membrane structure formed from a phospholipid having an acid value of 5 mg KOH/g or more.
[12] The skin improving agent according to [11], wherein the phospholipid is a hydrogenated phospholipid.
[13] The skin improving agent according to [11] or [12], wherein the lipid membrane structure is a single-layer lamellar structure.
[14] The skin improving agent according to [11] or [12], wherein an average hydrodynamic diameter of the lipid membrane structure is 200 nm or smaller.
[15] The skin improving agent according to [11] or [12], wherein the skin improving agent has a wrinkle improving effect.
[16] The skin improving agent according to [11] or [12], wherein the skin improving agent has an elasticity improving effect.
[17] The skin improving agent according to [11] or [12], wherein the skin improving agent has a radiance improving effect.
[18] The skin improving agent according to [11] or [12], wherein the skin improving agent has a texture improving effect.
[19] The skin improving agent according to [11] or [12], wherein the skin improving agent has a dullness improving effect.
[20] The skin improving agent according to [11] or [12], wherein the skin improving agent has a redness improving effect.
[21] The skin improving agent according to [11] or [12], wherein the skin improving agent has an itching improving effect.
[22] The skin improving agent according to [11] or [12], wherein the skin improving agent has a rough skin preventing effect.
[23] The skin improving agent according to [11] or [12], wherein the skin improving agent has a sagging improving effect.
[24] The skin improving agent according to [11] or [12], wherein the skin improving agent has a dark circle improving effect.

### Advantageous Effects of Invention

The present invention can provide a pore improving agent that safely prevents any one or more of pore-related problems such as pore blackhead, large pores or sagging pores, clogging by keratin plugs, and acnes, and also improves conditions of the problems, with less burden on the skin without requiring special treatment or drugs.

The present invention can also provide a skin improving agent that safely prevents rough skin and improves or prevents conditions of skin problems such as skin redness, dullness, and wrinkles, with less burden even on sensitive skin without requiring special treatment or drugs.

### Brief Description of Drawing

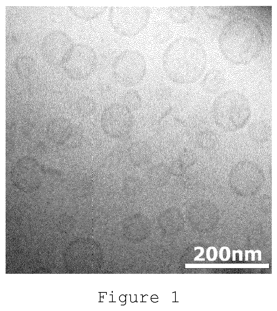

[Figure 1] Figure 1 is a photograph of lipid membrane structures formed in Example 5 and Example 12, observed by cryogenic transmission electron microscopy (Cryo-TEM).

### Description of Embodiments

Hereinafter, the mode for carrying out the present invention (hereinafter, also referred to as the "present embodiment") will be described in detail. The present invention is not limited by the present embodiment, and various changes or modifications can be made therein without departing from the spirit of the present invention.

The pore improving agent according to the first embodiment of the present embodiment is a pore improving agent comprising a composition containing a lipid membrane structure formed from a phospholipid having an acid value of 5 mg KOH/g or more (hereinafter, also referred to as a "lipid membrane structure-containing composition").

The skin improving agent according to the second embodiment of the present embodiment is a skin improving agent comprising a composition containing a lipid membrane structure formed from a phospholipid having an acid value of 5 mg KOH/g or more.

In the present specification, the "lipid membrane structure" means particles having a lamellar (lipid bilayer) structure in which lipid molecules are arranged with their hydrophilic groups facing outward and their hydrophobic groups facing each other. Specific examples of the form thereof include those described in the section <Lipid membrane structure-containing composition> mentioned later. In the present specification, it shall be understood that the lipid membrane structure has been formed if the average hydrodynamic diameter of a lipid membrane structure-containing composition prepared using a composition for lipid membrane structure formation can be measured using a dynamic light scattering measurement apparatus, more specifically, Zetasizer Nano ZSP (manufactured by Malvern Instruments Ltd.).

### <Composition for lipid membrane structure formation>

The composition for lipid membrane structure formation according to the present embodiment is a composition for obtaining a lipid membrane structure-containing composition. Hereinafter, components of the composition for lipid membrane structure formation will be described.

### [Component (A)]

The composition for lipid membrane structure formation according to the present embodiment comprises a phospholipid having an acid value of 5 mg KOH/g or more as a component (A). One or two or more phospholipids having an acid value of 5 mg KOH/g or more may be used, or one or more phospholipids having an acid value of less than 5 mg KOH/g and one or more phospholipids having an acid value of 5 mg KOH/g or more may be combined to attain the acid value of 5 mg KOH/g or more.

The origin of the phospholipid is not particularly limited, and lecithin is particularly preferred because lecithin is naturally derived and can be suitably used in cosmetics and external skin preparations. Lecithin may be derived from soybean, egg yolk, rapeseed, sunflower, corn, or the like and is preferably derived from a plant such as soybean, rapeseed, sunflower, or corn and more preferably derived from soybean because of easy availability and quality stability.

The phospholipid is preferably a hydrogenated phospholipid from the viewpoint of any one or more of prevention of oxidative deterioration ascribable to a heating step in cosmetic or quasi-drug production or oxidative deterioration during storage of such products, prevention of promotion of lipid oxidation in the stratum corneum and keratin plugs around pores to which the phospholipid or its lipid membrane structure is applied due to oxidative deterioration of the phospholipid or the lipid membrane structure, structural maintenance or enhancement of intercellular lipids (barrier function) in the stratum corneum when the applied lipid membrane structure permeates a deep part of the stratum corneum, and enhancement of the barrier function of the lipid membrane formed on the skin by the lipid membrane structure remaining on the surface of the stratum corneum. The hydrogenated phospholipid can be obtained by adding a hydrogen atom to an unsaturated carbon bond of a phospholipid by a conventional method known in the art. The hydrogenated phospholipid is particularly preferably hydrogenated lecithin.

The acid value of the phospholipid used in the present embodiment is 5 mg KOH/g or more. In the case of using a phospholipid having an acid value of less than 5 mg KOH/g, the dispersibility of the resulting lipid membrane structure is deteriorated so that a sufficient pore improving effect or skin improving effect cannot be obtained. The acid value of the phospholipid is preferably 6, 7, 8, 9, 10, or 11 mg KOH/g or more, more preferably 12, 13, or 14 mg KOH/g or more, further preferably 15 or 16 mg KOH/g or more, furthermore preferably 17, 18, or 19 mg KOH/g or more, particularly preferably 20 or 21 mg KOH/g or more, particularly more preferably 22, 23, 24, 25, 26, or 27 mg KOH/g or more, from the viewpoint of obtaining a finer lipid membrane structure. The upper limit of the acid value of the phospholipid is not particularly limited and is, for example, 70 mg KOH/g or less, 60 mg KOH/g or less, 50 mg KOH/g or less, or 40 mg KOH/g or less. The type of the phospholipid can be appropriately selected to obtain a phospholipid having a desired acid value. The acid value of the phospholipid shall be adopted as a value measured in accordance with "Japanese Standards of Quasi-drug Ingredients 2021, General Test Methods, Part 28. Acid Value Measurement Method".

A synthetic product or a commercially available product may be used as the phospholipid. Examples of the commercially available product include EMALEX SLP manufactured by Nihon Emulsion Co., Ltd. and SLP-White H manufactured by Tsuji Oil Mills Co., Ltd. One of these phospholipids may be used alone, or two or more thereof may be used in combination.

The content of the phospholipid in the composition for lipid membrane structure formation according to the present embodiment is not particularly limited, and the lower limit is preferably 5% by mass or more, more preferably 6, 7, 8, 9, 10, 11, 12, 13, 14, 15, 16, 17, 18, or 19% by mass or more. The upper limit is preferably 50% by mass or less, more preferably 45, 40, 35, 30, 25, or 20% by mass or less. When the content of the phospholipid is 5% by mass or more, the composition for lipid membrane structure formation efficiently forms a lipid membrane structure when dispersed in water. Thus, a sufficient pore improving effect or skin improving effect tends to be obtained. When the content is 50% by mass or less, the component (A) is more easily dissolved or dispersed in the composition for lipid membrane structure formation. Therefore, use of this composition for lipid membrane structure formation facilitates forming a lipid membrane structure excellent in dispersibility in an aqueous phase and tends to enhance the stability over time of the lipid membrane structure-containing composition.

### [Component (B)]

The composition for lipid membrane structure formation according to the present embodiment may comprise a compound represented by formula 1 given below as a component (B). The component (B) may be only one type of the compound or two or more types of the compounds.

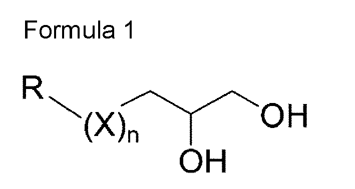

In the above formula 1, R is a substituted or unsubstituted alkyl group having 2 to 6 carbon atoms or a substituted or unsubstituted cycloalkyl group having 3 to 6 carbon atoms.

The alkyl group having 2 to 6 carbon atoms may be linear or branched. Examples of the alkyl group having 2 to 6 carbon atoms include an ethyl group, a n-propyl group, an isopropyl group, a n-butyl group, an isobutyl group, a sec-butyl group, a tert-butyl group, a n-pentyl group, an iso-amyl group, a tert-pentyl group, a neopentyl group, a n-hexyl group, a 3-methylpentan-2-yl group, a 3-methylpentan-3-yl group, a 4-methylpentyl group, a 4-methylpentan-2-yl group, a 1,3-dimethylbutyl group, a 3,3-dimethylbutyl group, and a 3,3-dimethylbutan-2-yl group. The alkyl group having 2 to 6 carbon atoms is preferably linear. Specifically, the alkyl group having 2 to 6 carbon atoms is preferably a group selected from the group consisting of an ethyl group, a n-propyl group, a n-butyl group, a n-pentyl group, and a n-hexyl group.

Examples of the cycloalkyl group having 3 to 6 carbon atoms include a cyclopropyl group, a cyclobutyl group, a cyclopentyl group, and a cyclohexyl group, with a cyclohexyl group being preferred.

The substituents for the alkyl group having 2 to 6 carbon atoms and the cycloalkyl group having 3 to 6 carbon atoms are not particularly limited without inhibiting the advantageous effects of the present invention. Examples of the substituents include halogen atoms, acyl groups, alkyl groups, aryl groups, alkoxyl groups, nitro groups, amino groups, and cyano groups. However, an alkyl group having 2 to 6 carbon atoms is not substituted by an alkyl group.

In the above formula 1, X is -O-, -C(=O)O-, or -O-C(=O)-, preferably -O-. In the above formula 1, n is 0 or 1. In the above formula 1, when X is -O- and n is 1, the number of carbon atoms in the alkyl group as R is preferably 4 or more.

The component (B) is preferably at least one selected from the group consisting of, for example, 1,2-pentanediol, 1,2-hexanediol, 1,2-heptanediol, 1,2-octanediol, cyclohexylglycerin, and hexylglycerin.

Particularly, in the case of using 1,2-hexanediol, 1,2-heptanediol, or hexylglycerin as the component (B), an exceedingly fine lipid membrane structure can be formed. In this respect, the lipid membrane structure is considered to form a bicelle. The bicelle has a disc-shaped single-layer lamellar structure and is therefore superior in permeability into the stratum corneum to a liposome having the same level of particle size thereas. Hence, at least one selected from the group consisting of 1,2-hexanediol, 1,2-heptanediol, and hexylglycerin is preferably used as the component (B) from the viewpoint of obtaining a lipid membrane structure with high permeability of a fat-soluble component.

The component (B) may be a synthetic product or a commercially available product.

The content of the component (B) in the composition for lipid membrane structure formation of the present embodiment is not particularly limited, and the lower limit is preferably 15% by mass or more, more preferably 20, 25, 30, 35, 40, 45, or 50% by mass or more. The upper limit is preferably 95% by mass or less, more preferably 90, 85, 80, 75, or 70% by mass or less, further preferably 65, 60, or 55% by mass or less.

In the composition for lipid membrane structure formation of the present embodiment, the content of the component (B) preferably exceeds 100 parts by mass with respect to 100 parts by mass of the component (A). When the content of the component (B) exceeds 100 parts by mass with respect to 100 parts by mass of the component (A), a fine lipid membrane structure tends to be easy to form. The content of the component (B) is preferably 110, 120, 130, 140, or 150 parts by mass or more with respect to 100 parts by mass of the component (A). When the content is 150 parts by mass or more, the component (A) is more easily dissolved or dispersed in the composition for lipid membrane structure formation. Therefore, use of this composition for lipid membrane structure formation tends to be able to form a lipid membrane structure excellent in dispersibility in an aqueous phase. The content of the component (B) is preferably 160, 170, 180, 190, or 200 parts by mass or more, more preferably 210, 220, 230, 240, or 250 parts by mass or more, further preferably 260, 270, 280, 290, or 300 parts by mass or more, furthermore preferably 310, 320, 330, 340, or 350 parts by mass or more, particularly preferably 360, 370, 380, 390, or 400 parts by mass or more, with respect to 100 parts by mass of the component (A) from the viewpoint of obtaining a finer lipid membrane structure. On the other hand, the upper limit of the content of the component (B) with respect to 100 parts by mass of the component (A) is not particularly limited. The content of the component (B) that exceeds 2000 parts by mass has less influence on the solubility or dispersibility of the component (A) in the composition for lipid membrane structure formation and is not economical. Therefore, the content is preferably 2000 parts by mass or less with respect to 100 parts by mass of the component (A). Thus, according to the present embodiment, the content of the component (B) is preferably more than 100 parts by mass and 2000 parts by mass or less with respect to 100 parts by mass of the component (A).

### [Component (C)]

The composition for lipid membrane structure formation of the present embodiment may further contain water as a component (C). In the case of using the component (A) having a high acid value, use thereof in combination with the component (B) and the component (C) can produce a composition for lipid membrane structure formation with better solubility or dispersibility of the component (A). Use of this composition for lipid membrane structure formation tends to be able to form a lipid membrane structure excellent in dispersibility in an aqueous phase. Furthermore, the component (C) plays a role in helping the blending of a component (D) (basic compound) and a component (E) (acidic compound) given below into the composition for lipid membrane structure formation. Specifically, the component (D) and/or the component (E) is dissolved in the component (C) in advance and then mixed with the component (A) and the component (B) so that the component (D) and/or the component (E) can be easily blended into the composition for lipid membrane structure formation.

The component (C) is preferably water with few impurities, for example, purified water that has been purified from ordinary water using a system that uses ion exchange, distillation, reverse osmosis, ultrafiltration and so on, either alone or in combination.

The content of the component (C) in the composition for lipid membrane structure formation of the present embodiment is not particularly limited, and the lower limit is preferably 0.5% by mass or more, more preferably 1, 2, 3, 4, or 5% by mass or more, further preferably 6, 7, 8, 9, 10, 11, 12, 13, 14, or 15% by mass or more, particularly preferably 16, 17, 18, 19, 20, 21, 22, 23, 24, or 25% by mass or more. The upper limit is preferably 75% by mass or less, more preferably 70, 65, or 60% by mass or less, further preferably 55, 50, 45, or 40% by mass or less, particularly preferably 35 or 30% by mass or less.

In the composition for lipid membrane structure formation of the present embodiment, the mass ratio of the component (B) to the component (C), (B)/(C), is preferably 0.3 to 100. The lower limit of the mass ratio range may be 0.3, 0.4, 0.5, 0.6, 0.7, 0.8, 0.9, or 1.0. The upper limit of the mass ratio range may be 100, 90, 80, 70, 60, 50, 40, 30, 20, 10, 9, 8, 7, or 6. The mass ratio range is, for example, more preferably 0.5 to 9, further preferably 1.0 to 6.

### [Component (D)]

The composition for lipid membrane structure formation of the present embodiment may further contain a basic compound as a component (D). In the case of using the component (A) having a high acid value, the composition for lipid membrane structure formation obtained so as to contain the component (D) can have better solubility or dispersibility of the component (A). Use of this composition for lipid membrane structure formation tends to be able to enhance the dispersibility of the lipid membrane structure in an aqueous phase. Only one type of component (D) may be used, or two or more types of compounds (D) may be used.

Examples of the component (D) include: inorganic bases, such as sodium hydroxide, potassium hydroxide, and ammonia; basic amino acids, such as arginine, lysine, and histidine; and amine compounds, such as ethanolamine, diethanolamine, triethanolamine, 2-amino-2-methyl-1,3-propanediol (AMPD), and 2-amino-2-hydroxymethyl-1,3-propanediol (tromethamine). Among them, arginine is preferred.

A synthetic product or a commercially available product may be used as the component (D).

The content of the component (D) in the composition for lipid membrane structure formation of the present embodiment is not particularly limited, and the lower limit is preferably 0.01% by mass or more, more preferably 0.02% by mass or more. The upper limit is preferably 2% by mass or less, more preferably 1% by mass or less.

In the composition for lipid membrane structure formation of the present embodiment, the lower limit of the content of the component (D) is preferably 0.05 parts by mass or more, more preferably 0.1 parts by mass or more, with respect to 100 parts by mass of the component (A). The upper limit is preferably 10 parts by mass or less, more preferably 5 parts by mass or less, with respect to 100 parts by mass of the component (A).

### [Component (E)]

The composition for lipid membrane structure formation of the present embodiment may contain an acidic compound as a component (E). The composition for lipid membrane structure formation obtained so as to contain the component (E) can have better solubility or dispersibility of the component (A). Use of this composition for lipid membrane structure formation tends to be able to enhance the dispersibility of the lipid membrane structure in an aqueous phase. Only one type of component (E) may be used, or two or more types of compounds (E) may be used.

Examples of the component (E) include: inorganic acids such as hydrochloric acid, sulfuric acid, nitric acid, phosphoric acid, and carbonic acid; and organic acids such as acetic acid, formic acid, propionic acid, butyric acid, citric acid, lactic acid, succinic acid, malic acid, tartaric acid, pyrrolidonecarboxylic acid (PCA), gluconic acid, benzoic acid, ethylenediaminetetraacetic acid (EDTA), etidronic acid, pentetic acid, and phytic acid. Among them, organic acids are preferred from the viewpoint that the resulting composition for lipid membrane structure formation can have better solubility or dispersibility of the component (A) .

Among them, the component (E) is preferably an organic acid having a chelating effect, from the viewpoint that the resulting composition for lipid membrane structure formation can have better solubility or dispersibility of the component (A), from the viewpoint of forming a lipid membrane structure excellent in dispersibility in an aqueous phase using the composition for lipid membrane structure formation, and from the viewpoint of enhancing the preservation stability of the composition for lipid membrane structure formation and the lipid membrane structure-containing composition obtained using the composition for lipid membrane structure formation. The component (A) may have the property of easily binding to metal ions. In this case, the organic acid having a chelating effect added as the component (E) captures metal ions in the composition for lipid membrane structure formation and enhances the preservation stability of the composition for lipid membrane structure formation while the component (A) is more easily dissolved or dispersed and the resulting composition for lipid membrane structure formation can have better solubility or dispersibility of the component (A). Use of this composition can presumably form a lipid membrane structure excellent in dispersibility in an aqueous phase and preservation stability. The organic acid having a chelating effect is preferably, for example, citric acid, ethylenediaminetetraacetic acid (EDTA), etidronic acid, or pentetic acid, more preferably ethylenediaminetetraacetic acid (EDTA).

A synthetic product or a commercially available product may be used as the component (E).

In the composition for lipid membrane structure formation of the present embodiment, the content (based on a free acid) of the component (E) is not particularly limited, and the lower limit is preferably 0.02% by mass or more, more preferably 0.04% by mass or more. The upper limit is preferably 4% by mass or less, more preferably 2% by mass or less.

In the composition for lipid membrane structure formation of the present embodiment, the lower limit of the content (based on a free acid) of the component (E) is preferably 0.1 parts by mass or more, more preferably 0.2 parts by mass or more, per 100 parts by mass of the component (A). The upper limit is more preferably 20 parts by mass or less, more preferably 10 parts by mass or less, per 100 parts by mass of the component (A).

The composition for lipid membrane structure formation of the present embodiment preferably comprises both the component (D) and the component (E) from the viewpoint of enhancing the solubility of the component (D) and the component (E) in the composition for lipid membrane structure formation and keeping the pH of the composition within a predetermined range during dispersion in an aqueous phase. Such a composition for lipid membrane structure formation may be obtained by separately adding the component (D) and the component (E) or may be obtained by adding a salt of the component (D) and the component (E).

Examples of the salt of the component (D) and the component (E) include, but are not particularly limited to: lysine hydrochloride; citric acid sodium salts such as trisodium citrate; phosphoric acid sodium salts such as disodium hydrogen phosphate; sodium benzoate; ethylenediaminetetraacetic acid sodium salts such as trisodium ethylenediaminetetraacetate (EDTA-3Na); and diethylenetriaminepentaacetic acid sodium salts such as pentasodium diethylenetriaminepentaacetate (5Na pentetate).

When the composition for lipid membrane structure formation contains the component (D) and the component (E), the mass ratio of the component (D) to the component (E), (D)/(E), is 0.2 to 10. The lower limit of the mass ratio range may be 0.3, 0.4, or 0.5. The upper limit of the mass ratio range may be 10, 9, 8, 7, 6, or 5. The mass ratio range is preferably, for example, 0.5 to 5.

In the composition for lipid membrane structure formation of the present embodiment, the lower limit of the total content of the component (D) and the component (E) is preferably 0.05% by mass or more, more preferably 0.1% by mass or more. The upper limit is preferably 4% by mass or less, more preferably 2% by mass or less.

### [Component (F)]

The composition for lipid membrane structure formation of the present embodiment may further contain a fat-soluble compound as a component (F). In the case of using the component (A) having a high acid value, use thereof in combination with the component (B) and the component (F) can produce a composition for lipid membrane structure formation with better solubility or dispersibility of the component (A). Use of this composition for lipid membrane structure formation tends to be able to form a lipid membrane structure having excellent dispersibility in an aqueous phase and high preservation stability. Furthermore, use of the component (F) in combination with the component (D) and/or the component (E) tends to be able to form a finer lipid membrane structure. Only one component (F) may be used, or two or more components (F) may be used.

Examples of the component (F) include: sterols, such as phytosterols, cholesterol, di(phytosteryl/octyldodecyl) lauroyl glutamate, phytosteryl oleate, and phytosteryl glucoside; triterpenes, such as γ-oryzanol, glycyrrhizinic acid, ursolic acid, and *Centella asiatica* extracts (mixtures of asiatic acid, madecassic acid, and asiaticoside); fat-soluble vitamins, such as retinol, hydrogenated retinol, cholecalciferol, tocopherol, and ascorbic acid ester; carotenoids, such as astaxanthin and β-carotene; coenzymes, such as ubiquinone; hydrocarbons, such as limonene, petrolatum, and squalane; ceramides, such as ceramide EOS, ceramide NG (ceramide 2), ceramide NP (ceramide 3), ceramide AP (ceramide 6II), ceramide EOP (ceramide 1), dihydroxylignocelloyl phytosphingosine, cerebroside, glycosphingolipid, and cetyl PG hydroxyethyl palmitamide; and polyphenols, such as tetrahydrodiferuloylmethane and pterostilbene. Among them, the component (F) is preferably at least one selected from the group consisting of phytosterols, cholesterol, γ-oryzanol, glycyrrhizinic acid, ursolic acid, a *Centella asiatica* extract (a mixture of asiatic acid, madecassic acid, and asiaticoside), hydrogenated retinol, tocopherol, and astaxanthin, ubiquinone, ceramide NG, ceramide NP, ceramide AP, ceramide EOP, tetrahydrodiferuloylmethane, and pterostilbene, from the viewpoint that the combination of the component (F) with the component (A) and the component (B) mutually enhances the solubility of the component (A) and the component (F), attains better solubility of the component (A), allows the component (F) to be easily blended, and can produce a composition for lipid membrane structure formation having good preservation stability, and from the viewpoint of enhancing the preservation stability of the lipid membrane structure-containing composition obtained using the composition for lipid membrane structure formation.

A synthetic product or a commercially available product may be used as the component (F). Examples of the commercially available product include Phytosterol-SKP manufactured by Tama Biochemical Co., Ltd., TECA manufactured by Bayer Holding Ltd., dl-α-tocopherol manufactured by DSM Corporation, NIKKOL(R) Retinol H10, NIKKOL(R) VC-IP, and squalane manufactured by Nikko Chemicals Co., Ltd., Astaxanthin-20C and γ-oryzanol manufactured by Oryza Yuka Co., Ltd., Kaneka Coenzyme Q10 manufactured by Kaneka Co., Ltd., CERAMIDE2 manufactured by Croda Japan Co., Ltd., and Ursolic Acid 90%, SabiWhite, and pTeroWhite manufactured by Sabinsa Japan Corporation.

The content of the component (F) in the composition for lipid membrane structure formation of the present invention is not particularly limited, and the lower limit is preferably 0.001% by mass or more, more preferably 0.01% by mass or more, further preferably 0.1% by mass or more, particularly preferably 1% by mass or more. The upper limit is preferably 12% by mass or less, more preferably 10% by mass or less, further preferably 8% by mass or less, particularly preferably 6% by mass or less.

In the composition for lipid membrane structure formation of the present embodiment, the lower limit of the content of the component (F) is preferably 0.005 parts by mass or more, more preferably 0.05 parts by mass or more, further preferably 0.5 parts by mass or more, particularly preferably 5 parts by mass or more, with respect to 100 parts by mass of the component (A). The upper limit is preferably 60 parts by mass or less, more preferably 50 parts by mass or less, further preferably 40 parts by mass or less, particularly preferably 30 parts by mass or less, with respect to 100 parts by mass of the component (A).

### [Other components]

The composition for lipid membrane structure formation of the present embodiment may further contain components other than the components (A) to (F) (other components). Examples of other components include, but are not particularly limited to, oil agents, surfactants, moisturizers, whitening agents, color materials, alcohols, amino acids, sugars, vitamins, viscosity modifiers, polymers, colorants, powders, ultraviolet absorbers, antiseptics, antimicrobial agents, antioxidants, perfumes, beauty ingredients, electrolytes, fibers, and plant extracts. One of these components may be used alone, or two or more thereof may be used in combination. For example, beauty ingredients, such as a moisturizer and a whitening agent are blended into the composition for lipid membrane structure formation, and this composition for lipid membrane structure formation can be dispersed in an aqueous phase to form a lipid membrane structure including the beauty ingredients. For forming the lipid membrane structure including the beauty ingredients, the timing of blending the beauty ingredients is not particularly limited. For example, a composition for lipid membrane structure formation containing components other than the beauty ingredients is prepared and then stored. In preparing the lipid membrane structure-containing composition, the beauty ingredients are added to the composition for lipid membrane structure formation and uniformly dissolved therein, and the resulting composition may then be dispersed in an aqueous phase to form a lipid membrane structure including the beauty ingredients. In this respect, the composition for lipid membrane structure formation not only simplifies the production of each individual pore improving agent or skin improving agent, but also enables different product groups to be conveniently created by stocking the composition for lipid membrane structure formation beforehand and thereby arbitrarily changing the beauty ingredients included therein.

The pH of the composition for lipid membrane structure formation dispersed in an aqueous phase is preferably 4.0 or higher, more preferably 5.0 or higher, further preferably 7.0 or higher, furthermore preferably 8.0 or higher, from the viewpoint of obtaining a finer lipid membrane structure. On the other hand, the pH of the composition for lipid membrane structure formation dispersed in an aqueous phase is preferably 10.0 or lower, more preferably 9.5 or lower, furthermore preferably 9.0 or lower, from the viewpoint of obtaining a lipid membrane structure having a uniform particle size (i.e., a highly homogeneous lipid membrane structure-containing composition). Specifically, the pH of the composition for lipid membrane structure formation of the present embodiment dispersed in an aqueous phase is preferably 4.0 to 10.0. In this context, the "pH of the composition for lipid membrane structure formation dispersed in an aqueous phase" means the pH of a product obtained by dispersing the composition for lipid membrane structure formation in the aqueous phase and is measured by the following method.

To a 200 mL beaker, 100 mL of purified water is added to prepare an aqueous phase. The aqueous phase is warmed to 80°C, and the composition for lipid membrane structure formation of 80°C prepared as described above is added thereto with stirring at 100 rpm. After the completion of addition, the mixture is stirred at 80°C for 2 minutes to prepare a lipid membrane structure-containing composition. The lipid membrane structure-containing composition thus prepared is spontaneously cooled to room temperature (25°C), and the pH at 25°C is measured using a pH meter based on a glass electrode method (HM-25R, manufactured by DKK Toa Co., Ltd.).

### <Lipid membrane structure-containing composition>

The lipid membrane structure-containing composition according to the present embodiment is a composition containing a lipid membrane structure formed from a phospholipid having an acid value of 5 mg KOH/g or more and can be obtained using the composition for lipid membrane structure formation mentioned above. However, the composition containing a lipid membrane structure formed from a phospholipid having an acid value of 5 mg KOH/g or more can exert effects as the pore improving agent or the skin improving agent according to the present embodiment. Therefore, a production method therefor is not particularly limited, and a known method can be used. For example, a fine single-layer lamellar structure may be prepared by use of sonication, ethanol injection, or cholic acid removal, or a fine lipid membrane structure may be prepared by combining a highpressure emulsifier such as a microfluidizer or a micronization approach such as extrusion with a method for preparing a lipid membrane structure containing a multilayer lamellar structure, such as the Bangham method or a polyhydric alcohol method. In forming the lipid membrane structure by use of such a known method, each individual production method using suitable components and steps can be performed without being limited by the configuration of the composition for lipid membrane structure formation mentioned above except that a phospholipid having an acid value of 5 mg KOH/g or more is contained as a component constituting the lipid membrane structure.

The form of the lipid membrane structure is not particularly limited as long as the lipid membrane structure has a lamellar (lipid bilayer) structure in which lipid molecules are arranged with their hydrophilic groups facing outward and their hydrophobic groups facing inward. Examples thereof include liposomes, bicelles, and α-gels. The lipid membrane structure may be a single-layer lamellar structure called unilamellar or single lamellar structure, may be a lamellar structure with several layers (2 to 10 layers) called oligolamellar structure, may be a multilamellar structure having a larger number of layers, or may be a mixture thereof. The lipid membrane structure is preferably a single-layer lamellar structure. The single-layer lamellar structure has a fine particle size and is also excellent in compound inclusion efficiency and permeability. It can be confirmed that the lipid membrane structure is a single-layer lamellar structure using, for example, cryogenic transmission electron microscopy (Cryo-TEM). In the present embodiment, the lipid membrane structure is preferably a unilamellar liposome. The unilamellar liposome has a fine particle size and a high internal aqueous phase volume and is therefore excellent in inclusion efficiency and permeability of water-soluble compounds. Hence, the unilamellar liposome is useful in a pore improving agent or a skin improving agent blended with water-soluble beauty ingredients. In the present embodiment, the lipid membrane structure is preferably a bicelle. The bicelle is a fine disc-shaped single-layer lamellar structure having a thickness of 3 to 10 nm and a diameter of 15 to 100 nm and is excellent in inclusion efficiency and permeability of fat-soluble components. Hence, the bicelle is useful in a pore improving agent or a skin improving agent blended with fat-soluble beauty ingredients. In the lipid membrane structure-containing composition according to the present embodiment, only any one of the unilamellar liposome and the bicelle may be present, or a mixture of these two structures may be present.

The upper limit of the average hydrodynamic diameter of the lipid membrane structure is preferably 200 nm or smaller, more preferably 190, 180, 170, 160, 150, 140, 130, 120, 110, 100, 90, or 80 nm or smaller, from the viewpoint of dispersibility, preservation stability, and permeability into the skin. The lower limit of the average hydrodynamic diameter of the lipid membrane structure is not particularly limited and is, for example, 20 nm or larger from the viewpoint of single-layer lamellar structure formation efficiency.

The upper limit of the polydispersity index (PDI) of the lipid membrane structure is preferably 0.80 or less, more preferably 0.50 or less, further preferably 0.40 or less, furthermore preferably 0.30 or less, still further preferably 0.25 or less, particularly preferably 0.20 or less, from the viewpoint of dispersibility, preservation stability, and permeability into the skin. The lower limit of the polydispersity index (PDI) of the lipid membrane structure is not particularly limited and is, for example, 0.01 or more.

The average hydrodynamic diameter and the polydispersity index (PDI) of the lipid membrane structure shall be adopted as a harmonic average diameter (Z-Average) and a polydispersity index (PDI) based on scattered light intensity by cumulant analysis using a dynamic light scattering measurement apparatus (manufactured by Malvern Instruments Ltd., Zetasizer Nano ZSP). The average hydrodynamic diameter and the polydispersity index used herein shall be defined in accordance with description in "JIS Z8828: 2019 Particle Size Analysis - Dynamic Light Scattering Method".

The concentration of the lipid membrane structure in the lipid membrane structure-containing composition is preferably 0.0001% by mass or more, more preferably 0.01% by mass or more, further preferably 1% by mass or more, from the viewpoint of obtaining a sufficient pore improving effect or skin improving effect. On the other hand, the upper limit of the concentration is not particularly limited and is, for example, less than 5% by mass.

The pH of the lipid membrane structure-containing composition is preferably 4.0 to 10.0. Specifically, the pH shall be adopted as pH measured using a pH meter based on a glass electrode method (HM-25R manufactured by DKK Toa Co., Ltd.).

### <Method for producing lipid membrane structure-containing composition>

A method for producing the lipid membrane structure-containing composition of the present invention is not particularly limited. In forming the lipid membrane structure by use of a known method, each individual production method using suitable components and steps can be performed except that a phospholipid having an acid value of 5 mg KOH/g or more is contained as a component constituting the lipid membrane structure. In the case of using the composition for lipid membrane structure formation mentioned above, a method of mixing the component (A) described above and optionally the components (B) to (F) and other components and adding a large excess of water to the resulting composition for lipid membrane structure formation may be used, or a method of adding the composition for lipid membrane structure formation to a large excess of water may be used. The latter method is preferred from the viewpoint of improving the dispersibility of the lipid membrane structure. Specifically, the method for producing the lipid membrane structure-containing composition according to the present embodiment preferably involves mixing the component (A) described above and optionally the components (B) to (F) and other components and dispersing the resulting composition for lipid membrane structure formation in an aqueous phase.

More specifically, the method for producing the lipid membrane structure-containing composition according to the present embodiment preferably comprises the steps of: mixing the component (A) described above and optionally one or more components selected from the group consisting of the component (B), the component (C), the component (D), the component (E), the component (F), and other components to obtain a composition for lipid membrane structure formation (mixing step); and dispersing the composition for lipid membrane structure formation in an aqueous phase (dispersion step).

### (Mixing step)

In the mixing step, the individual components may be mixed at once or may be sequentially mixed. In the case of sequentially mixing the individual components, the order thereof is not particularly limited. For example, in the case of preparing the composition for lipid membrane structure formation using the component (A), the component (B), and the component (C), the component (A) and the component (B) may be mixed and then supplemented and mixed with the component (C); the component (A) and the component (C) may be mixed and then supplemented and mixed with the component (B); or the component (B) and the component (C) may be added at the same time (e.g., a mixed solvent of the component (B) and the component (C) may be added) to the component (A) and mixed. For example, in the case of preparing the composition for lipid membrane structure formation using the component (C), the component (D), and/or the component (E) in addition to the component (A) and the component (B), the component (C), the component (D), and/or the component (E) may be separately added or may be mixed in advance and added as a mixture. For example, in the case of preparing the composition for lipid membrane structure formation using the component (C), the component (D), and/or the component (E) in addition to the component (A), the component (B), and the component (F), the order of addition of the component (F) is not particularly limited. For example, the component (A), the component (B), and the component (F) may be mixed and then supplemented with the component (C), the component (D), and/or the component (E) either at the same time or sequentially. Alternatively, the component (A) and the component (B) as well as the component (C), the component (D), and/or the component (E) may be mixed either at the same time or sequentially and then finally supplemented with the component (F). In the case of preparing the composition for lipid membrane structure formation further using a component described in the section [Other components], the order of addition of the component described in the section [Other components] is not particularly limited and may be appropriately selected according to, for example, solubility. For example, a fat-soluble component may be added together with the addition of the component (F) or may be finally added. Alternatively, a water-soluble component may be added together with the addition of the component (C), the component (D), and/or the component (E) or may be finally added.

The mixing temperature in mixing the individual components is not particularly limited and is preferably equal to or higher than the phase transition temperature of the component (A). For example, the upper limit is 120, 110, 100, 95, or 90°C, and the lower limit is 40, 45, 50, 55, 60, 65, 70, 75, 80, or 85°C. The mixing temperature is, for example, 40 to 120°C, preferably 40 to 100°C, more preferably 60 to 90°C. The mixing time is not particularly limited either and is preferably 10 to 180 minutes. The mixing method is not particularly limited and can be performed using a mixing approach known in the art such as a magnetic stirrer (e.g., a hot stirrer), a paddle mixer, a propeller mixer, or a planetary mixer because a high degree of mechanical shearing force is not required.

In the mixing step, other steps such as purification (e.g., filtration), cooling, and storage may be appropriately performed in addition to the mixing step of the individual components described above. For example, a mixture is prepared by mixing the component (A) and the component (B) and optionally the components (C) to (F) and a portion of other components, and undissolved substances contained in the mixture are filtered off. After cooling and storage, in preparing the lipid membrane structure-containing composition, the mixture may be blended, if necessary, with the remaining components (B) to (F) and other components to obtain a composition for lipid membrane structure formation.

### (Dispersion step)

The composition for lipid membrane structure formation obtained by the mixing step may be dispersed in an aqueous phase by addition (of the composition for lipid membrane structure formation to the aqueous phase) without stirring of the aqueous phase, with stirring of the aqueous phase, or by addition of the aqueous phase to the composition for lipid membrane structure formation, because the composition for lipid membrane structure formation spontaneously forms a fine lipid membrane structure without substantial mechanical shearing force. In this respect, the stirring conditions for the aqueous phase and/or the composition for lipid membrane structure formation are not particularly limited, and the stirring is performed, for example, using a stirring approach known in the art at a rotation speed of 10 to 300 rpm. The composition for lipid membrane structure formation and/or the aqueous phase may be added at once, may be added in divided portions, or may be sequentially added at an arbitrary addition rate using a dropwise addition approach known in the art.

In the aqueous phase, water with few impurities is preferably used. For example, purified water that has been purified from ordinary water using a system that uses ion exchange, distillation, reverse osmosis, or ultrafiltration, either alone or in combination, is preferably used. The aqueous phase may further contain components other than water as long as the lipid membrane structure can be formed. Examples of the components other than water include oil agents, surfactants, moisturizers, whitening agents, color materials, alcohols, amino acids, sugars, vitamins, viscosity modifiers, polymers, colorants, powders, ultraviolet absorbers, antiseptics, antimicrobial agents, antioxidants, perfumes, beauty ingredients, electrolytes, fibers, and plant extracts.

In the dispersion step, the temperature of the aqueous phase is preferably equal to or higher than the phase transition temperature of the component (A) because the lipid membrane structure tends to be efficiently formed. For example, the upper limit of the temperature of the aqueous phase is 100, 95, or 90°C, and the lower limit is 0, 5, 10, 15, 20, 25, 30, 35, 40, 45, 50, 55, or 60°C. The temperature of the aqueous phase is, for example, 0 to 100°C, preferably 25 to 100°C, more preferably 40 to 95°C, furthermore preferably 60 to 90°C.

In the dispersion step, the temperature of the composition for lipid membrane structure formation to be dispersed is preferably equal to or higher than the phase transition temperature of the component (A) because the lipid membrane structure tends to be efficiently formed. For example, the upper limit of the temperature of the composition for lipid membrane structure formation is 120, 110, 100, 95, or 90°C, and the lower limit is 0, 5, 10, 15, 20, 25, 30, 35, 40, 45, 50, 55, or 60°C. The temperature of the composition for lipid membrane structure formation is, for example, 0 to 120°C, preferably 25 to 120°C, more preferably 40 to 100°C, furthermore preferably 60 to 90°C.

In the method for producing the lipid membrane structure-containing composition according to the present embodiment, other steps such as purification (e.g., filtration), cooling, and storage may be further performed in addition to the mixing step and the dispersion step.

### <Pore improving agent and skin improving agent>

The lipid membrane structure-containing composition of the present embodiment may be used as it is as a pore improving agent or a skin improving agent, or the lipid membrane structure-containing composition may be blended into a cosmetic, an external skin preparation, or a skin improving agent and thereby impart a pore improving effect or a skin improving effect thereto.

The form of the pore improving agent or the skin improving agent of the present embodiment is not particularly limited as long as the lipid membrane structure is stably blended thereinto. Examples thereof include lotions, gels, emulsions, creams, shampoos, and face washes. A blend with a low viscous lotion, which is difficult to blend with the lipid membrane structure, is preferred from the viewpoint of exploiting the transparency of appearance, the feeling of permeation upon application, and high stability brought about by containing the fine (e.g., an average hydrodynamic diameter of 200 nm or smaller) lipid membrane structure of the present embodiment.

The pore improving agent or the skin improving agent of the present embodiment may be further blended with a component that is usually used in cosmetics or external skin preparations, in addition to the lipid membrane structure-containing composition described above, without impairing the advantageous effects of the present invention. Examples of such a component include, but are not limited to, oil agents, surfactants, moisturizers, whitening agents, color materials, alcohols, amino acids, vitamins, viscosity modifiers, polymers, colorants, powders, ultraviolet absorbers, antiseptics, antimicrobial agents, antioxidants, perfumes, beauty ingredients, electrolytes, pH adjusters, fibers, water, and plant extracts.

The concentration of the lipid membrane structure in the pore improving agent or the skin improving agent prepared by blending the lipid membrane structure-containing composition of the present embodiment is preferably 0.0001% by mass or more, more preferably 0.01% by mass or more, further preferably 1% by mass or more. On the other hand, the upper limit of the concentration is not particularly limited and is, for example, less than 5% by mass.

The pore improving effect according to the first embodiment is not particularly limited as long as pore-related problems are improved. Examples thereof include a pore tightening effect on pores visible in shape such as large pores or sagging pores, a sebum secretion regulating effect, an effect of surfacing or shedding keratin plugs or an effect of suppressing keratin plug formation, or both, an effect of soothing acnes or an effect of suppressing acne development, or both, a pore blackhead improving effect, and an effect of improving makeup adherence or an effect of improving makeup durability, or both.

The skin improving effect according to the second embodiment is not particularly limited as long as skinrelated problems are improved. Examples thereof include any one or more effect, such as a wrinkle improving effect, an elasticity improving effect, a radiance improving effect, a texture improving effect, a dullness improving effect, a redness improving effect, an itching improving effect, a rough skin (intrinsic due to menstrual cycle) preventing effect, a rough skin (extrinsic due to seasonal change, pollen, wearing a mask, etc.) preventing effect, a sagging improving effect, and a dark circle improving effect.

The pore improving effect and the skin improving effect also include an immediate effect or a continuous effect, or both. The immediate effect refers to, for example, an effect that starts to appear 1 to 30 minutes, preferably 3 to 20 minutes, and more preferably 5 to 15 minutes, after application of the pore improving agent or the skin improving agent. The continuous effect refers to, for example, an effect that starts to appear after 1 day to 1 month, preferably 3 days to 3 weeks, more preferably 5 days to 2 weeks, while the pore improving agent or the skin improving agent is applied to the skin twice in the morning and evening, and is maintained or more enhanced while the application is continued thereafter.

### Example 1

The present invention will be described further specifically with reference to Examples and Comparative Examples. However, the present invention is not limited by these Examples, etc. by any means. In Examples given below, various operations were performed at room temperature (20 to 25°C) unless otherwise specified. "%" and "parts" mean "% by mass" and "parts by mass", respectively, unless otherwise specified.

### <Preparation of lipid membrane structure-containing composition>

A lipid membrane structure-containing composition was prepared by the following method. For lecithin, commercially available hydrogenated and unhydrogenated lecithins derived from soybean having different acid values (acid value: 0.3 to 30.8 mg KOH/g) were obtained and mixed at a ratio to attain the desired acid value. The following six hydrogenated and unhydrogenated lecithins derived from soybean were used.
Hydrogenated lecithin having an acid value of 0.3 mg KOH/g
Hydrogenated lecithin having an acid value of 6.1 mg KOH/g
Hydrogenated lecithin having an acid value of 15.0 mg KOH/g
Hydrogenated lecithin having an acid value of 20.0 mg KOH/g
Hydrogenated lecithin having an acid value of 23.1 mg KOH/g
Unhydrogenated lecithin having an acid value of 17.0 mg KOH/g

### [Examples 1 to 14 and Comparative Examples 1 to 4]

The components (A) to (F) shown in Tables 1 and 2 (unit of content; parts by mass) were heated to 80°C and uniformly mixed to obtain a composition for lipid membrane structure formation. The composition for lipid membrane structure formation described above was added to a component (G) heated to 80°C, uniformly mixed, and then cooled to obtain a lipid membrane structure-containing composition.

In Comparative Example 2 and Comparative Example 4, immiscible oil droplets were present at the time of mixing the components (A) to (F), and a uniform solution was not obtained. A composition obtained by mixing this into an aqueous phase had cloudy appearance and was not able to be confirmed to form a lipid membrane structure.

### [Appearance]

The appearance of the lipid membrane structure-containing composition prepared in each of Examples and Comparative Examples was visually observed at 25°C in a glass bottle container having a diameter of 3.7 cm, and evaluated on the basis of the following criteria (as for ⊚ or ○, it can be confirmed that the dispersibility of the lipid membrane structure is favorable):
⊚: Transparent to translucent liquid with visible letters on the opposite side of the container.
O: Homogeneous liquid, though letters on the opposite side of the container are not visible.
×: Separation or precipitation is seen.

### [Presence or absence of lipid membrane structure formation]

The lipid membrane structure-containing composition prepared in each of Examples and Comparative Examples was observed by Cryo-TEM using a transmission electron microscope (H-7650 manufactured by Hitachi High-Tech Corp.) and evaluated for the presence or absence of lipid membrane structure formation on the basis of the following criteria (as for ⊚ or ○, it can be confirmed that a lipid membrane structure is formed):
⊚: A unilamellar liposome structure or a bicelle structure is observed.
O: A multilamellar liposome structure is observed in addition to a unilamellar liposome structure or a bicelle structure.
×: No lipid membrane structure is observed.

### [Average hydrodynamic diameter and polydispersity index (PDI)]

The average hydrodynamic diameter and the polydispersity index (PDI) of the lipid membrane structure were measured as to the lipid membrane structure-containing composition prepared as described above by cumulant analysis using a dynamic light scattering measurement apparatus (manufactured by Malvern Instruments Ltd., Zetasizer Nano ZSP).

Figure 1 shows a Cryo-TEM image of the observed lipid membrane structure-containing compositions obtained in Example 5 and Example 12. The ring-shaped image shows a unilamellar liposome structure, and the bar-shaped image shows a bicelle structure. The formation of a lipid membrane structure having a single-layer lamellar structure can be confirmed.

### [Pore improving effect test]

The lipid membrane structure-containing composition obtained in each of Examples and Comparative Examples was used as a sample and subjected to effect tests a to k given below by 20 expert panelists (women in their 20s to 50s), and its effect compared to before the start of application was determined by sensory evaluation on the basis of the following criteria.
⊚: Very effective
○: Effective
△: Not changed
×: Condition worsens
a [Immediate pore tightening effect]

Each sample was applied to the skin and evaluated for its pore tightening effect 10 minutes later.

### b [Continuous pore tightening effect]

Each sample was applied to the skin twice in the morning and evening and evaluated for its pore tightening effect 1 week later.

### c [Spontaneous keratin plug surfacing or shedding effect]

Each sample was applied to the skin twice in the morning and evening and evaluated for its spontaneous keratin plug surfacing or shedding effect within 1 week.

### d [Sebum secretion regulating effect]

Each sample was applied to the skin at night and evaluated for its sebum secretion regulating effect at the time of awakening in the next morning.

### e [Keratin plug formation suppressing effect]

Each sample was applied to the skin twice in the morning and evening and evaluated for its keratin plug formation suppressing effect 1 week later.

### f [Pore blackhead improving effect]

Each sample was applied to the skin twice in the morning and evening and evaluated for its pore blackhead improving effect 1 week later.

### g [Immediate makeup adherence improving effect]

Each sample was applied to the skin in the morning and evaluated for its makeup adherence improving effect in putting makeup on.

### h [Continuous makeup adherence improving effect]

Each sample was applied to the skin twice in the morning and evening and evaluated for its makeup adherence improving effect in putting makeup on 1 week later.

### i [Makeup durability improving effect]

Each sample was applied to the skin in the morning and evaluated for its makeup durability improving effect in the daytime.

### j [Acne soothing effect]

Each sample was applied to an acned area twice in the morning and evening and evaluated for its spontaneous acne soothing effect within 1 week.

### k [Acne development suppressing effect]

Each sample was applied to the skin twice in the morning and evening and evaluated for its effect of reducing the development frequency of acnes for 4 weeks after the start of application.

### [Skin improving effect test]

The lipid membrane structure-containing composition obtained in each of Examples and Comparative Examples was used as a sample and subjected to effect tests a to p given below by 20 expert panelists (women in their 20s to 50s), and its effect compared to before the start of application was determined by sensory evaluation on the basis of the following criteria.
⊚: Very effective
○: Effective
△: Not changed
×: Condition worsens
a [Immediate wrinkle improving effect]

Each sample was applied to the skin and evaluated for its wrinkle improving effect 10 minutes later.

### b [Continuous wrinkle improving effect]

Each sample was applied to the skin twice in the morning and evening and evaluated for its wrinkle improving effect 1 week later.

### c [Immediate elasticity improving effect]

Each sample was applied to the skin and evaluated for its elasticity improving effect 10 minutes later.

### d [Continuous elasticity improving effect]

Each sample was applied to the skin twice in the morning and evening and evaluated for its elasticity improving effect 1 week later.

### e [Immediate radiance improving effect]

Each sample was applied to the skin and evaluated for its radiance improving effect 10 minutes later.

### f [Continuous radiance improving effect]

Each sample was applied to the skin twice in the morning and evening and evaluated for its radiance improving effect 1 week later.

### g [Immediate texture improving effect]

Each sample was applied to the skin and evaluated for its texture improving effect 10 minutes later.

### h [Continuous texture improving effect]

Each sample was applied to the skin twice in the morning and evening and evaluated for its texture improving effect 1 week later.

### i [Immediate dullness improving effect]

Each sample was applied to the skin and evaluated for its dullness improving effect 10 minutes later.

### j [Continuous dullness improving effect]

Each sample was applied to the skin twice in the morning and evening and evaluated for its dullness improving effect 1 week later.

### k [Redness improving effect]

Each sample was applied to a reddish area twice in the morning and evening and evaluated for its redness improving effect within 1 week.

### l [Itching improving effect]

Each sample was applied to an itchy area twice in the morning and evening and evaluated for its itching improving effect within 1 week.

### m [Intrinsic rough skin preventing effect]

Each sample was applied to the skin twice in the morning and evening and evaluated for its effect of preventing rough skin attributed to a menstrual cycle for 4 weeks after the start of application.

### n [Extrinsic rough skin preventing effect]

Each sample was applied to the skin twice in the morning and evening and evaluated for its effect of preventing rough skin attributed to wearing a mask for 4 weeks after the start of application.

### o [Sagging improving effect]

Each sample was applied to the skin twice in the morning and evening and evaluated for its sagging improving effect for 4 weeks after the start of application.

### p [Dark circle improving effect]

Each sample was applied to the skin twice in the morning and evening and evaluated for its dark circle improving effect for 4 weeks after the start of application.

The results of each evaluation described above are shown in Tables 1 and 2 below.

**[Table 1]**

| | Component | Starting material | Example 1 | Example 2 | Example 3 | Example 4 | Example 5 | Example 6 | Example 7 | Comparative Example 1 | Comparative Example 2 |
|---|---|---|---|---|---|---|---|---|---|---|---|
| | A' | Hydrogenated lecithin (acid value:0.3) | | | | | | | | 1.00 | |
| | A | Hydrogenated lecithin (acid value:6.1) | 1.00 | | | | | | | | |
| Composition for lipid membrane structure formation | | Hydrogenated lecithin (acid value:15.0) | | 1.00 | | | | | | | |
| | | Hydrogenated lecithin (acid value:20.0) | | | 1.00 | | | | | | |
| | | Hydrogenated lecithin (acid value:23.1) | | | | 1.00 | 1.00 | 1.00 | | | 1.00 |
| | | Unhydrogenated lecithin (acid value17.0) | | | | | | | 1.00 | | |
| | B | 1,2-Pentanediol | 4.00 | 4.00 | 4.00 | 4.00 | 4.00 | 2.00 | 4.00 | 4.00 | 4.00 |
| | C | Water | | | | | 1.49 | | | | |
| | D | Arginine | | | | | 0.03 | | | | |
| | E | Citric acid | | | | | 0.01 | | | | |
| | F | Phytosterols | | | | | 0.13 | | | | |
| | | Squalane | | | | | | | | | 2.00 |
| Aqueous phase for dispersion | G | Water | 95.00 | 95.00 | 95.00 | 95.00 | 93.34 | 97.00 | 95.00 | 95.00 | 93.00 |
| | Total | | 100.00 | 100.00 | 100.00 | 100.00 | 100.00 | 100.00 | 100.00 | 100.00 | 100.00 |
| | Appearance | | ⊚ | ⊚ | ⊚ | ⊚ | ⊚ | ⊚ | ⊚ | × | × |
| | Presence or absence of lipid membrane structure formation | | ⊚ | ⊚ | ⊚ | ⊚ | ⊚ | ⊚ | ⊚ | ⊚ | × |
| | Average hydrodynamic diameter (nm) | | 88 | 59 | 49 | 42 | 77 | 109 | 102 | 864 | Not measurable (1 µm or larger) |
| | Polydispersity index PDI | | 0.16 | 0.18 | 0.19 | 0.19 | 0.15 | 0.25 | 0.22 | 0.26 | Not measurable |
| Lipid membrane structure-containing composition | | Immediate pore tightening effect | ○ | ⊚ | ⊚ | ⊚ | ⊚ | ○ | ○ | △ | △ |
| | | Continuous pore tightening effect | ⊚ | ⊚ | ⊚ | ⊚ | ⊚ | ○ | ⊚ | △ | △ |
| | | Spontaneous keratin plug surfacing or shedding effect | ○ | ⊚ | ⊚ | ⊚ | ⊚ | ○ | ○ | △ | △ |
| | | Sebum secretion regulating effect | ○ | ⊚ | ⊚ | ⊚ | ⊚ | ○ | ○ | △ | △ |
| | Pore improving effect | Keratin plug formation suppressing effect | ⊚ | ⊚ | ⊚ | ⊚ | ⊚ | ○ | ⊚ | △ | △ |
| | | Pore blackhead improving effect | ○ | ⊚ | ⊚ | ⊚ | ⊚ | ○ | ○ | △ | △ |
| | | Immediate makeup adherence improving effect | ○ | ⊚ | ⊚ | ⊚ | ⊚ | ○ | ○ | △ | △ |
| | | Continuous makeup adherence improving effect | ⊚ | ⊚ | ⊚ | ⊚ | ⊚ | ○ | ⊚ | △ | △ |
| | | Makeup durability improving effect | ⊚ | ⊚ | ⊚ | ⊚ | ⊚ | ○ | ⊚ | △ | △ |
| | | Acne soothing effect | ○ | ⊚ | ⊚ | ⊚ | ⊚ | ○ | ○ | △ | △ |
| | | Acne development suppressing effect | ○ | ⊚ | ⊚ | ⊚ | ⊚ | ○ | ○ | △ | △ |

As shown in Table 1, the compositions of Examples 1 to 7 in which lipid membrane structures formed from hydrogenated and unhydrogenated lecithins having an acid value of 5 mg KOH/g or more were dispersed were found to have pore improving effects such as an immediate pore tightening effect, a continuous pore tightening effect, a spontaneous keratin plug surfacing or shedding effect, a sebum secretion regulating effect, a keratin plug formation suppressing effect, a pore blackhead improving effect, an immediate makeup adherence improving effect, a continuous makeup adherence improving effect, a makeup durability improving effect, an acne soothing effect, and an acne development suppressing effect. By contrast, in Comparative Example 1 using hydrogenated lecithin having an acid value of less than 5 mg KOH/g, the pore improving effects were insufficient, though the formation of a lipid membrane structure was able to be confirmed. In Comparative Example 2, albeit using hydrogenated lecithin having an acid value of 5 mg KOH/g or more, an O/W emulsion, not a lipid membrane structure, was formed due to blending of an excess of squalane, and the pore improving effects were insufficient.

**[Table 2]**

| | Component | Starting material | Example 8 | Example 9 | Example 10 | Example 11 | Example 12 | Example 13 | Example 14 | Comparative Example 3 | Comparative Example 4 |
|---|---|---|---|---|---|---|---|---|---|---|---|
| | A' | Hydrogenated lecithin (acid value:0.3) | | | | | | | | 1.00 | |
| | A | Hydrogenated lecithin (acid value:6.1) | 1.00 | | | | | | | | |
| | | Hydrogenated lecithin (acid value:15.0) | | 1.00 | | | | | | | |
| Composition for lipid membrane structure formation | | Hydrogenated lecithin (acid value:20.0) | | | 1.00 | | | | | | |
| | | Hydrogenated lecithin (acid value:23.1) | | | | 1.00 | 1.00 | 1.00 | | | 1.00 |
| | | Unhydrogenated lecithin (acid value:17.0) | | | | | | | 1.00 | | |
| | B | 1,2-Pentanediol | 4.00 | 4.00 | 4.00 | 4.00 | 4.00 | 2.00 | 4.00 | 4.00 | 4.00 |
| | C | Water | | | | | 1.49 | | | | |
| | D | Arginine | | | | | 0.03 | | | | |
| | E | Citric acid | | | | | 0.01 | | | | |
| | F | Phytosterols | | | | | 0.13 | | | | |
| | | Squalane | | | | | | | | | 2.00 |
| Aqueous phase for dispersion | G | Water | 95.00 | 95.00 | 95.00 | 95.00 | 93.34 | 97.00 | 95.00 | 95.00 | 93.00 |
| | Total | | 100.00 | 100.00 | 100.00 | 100.00 | 100.00 | 100.00 | 100.00 | 100.00 | 100.00 |
| | Appearance | | ⊚ | ⊚ | ⊚ | ⊚ | ⊚ | ⊚ | ⊚ | × | × |
| | Presence or absence of lipid membrane structure formation | | ⊚ | ⊚ | ⊚ | ⊚ | ⊚ | ⊚ | ⊚ | ⊚ | × |
| | Average hydrodynamic diameter (nm) | | 88 | 59 | 49 | 42 | 77 | 109 | 102 | 864 | Not measurable (1 µm or larger) |
| | Polydispersity index PDI | | 0.16 | 0.18 | 0.19 | 0.19 | 0.15 | 0.25 | 0.22 | 0.26 | Not measurable |
| Lipid membrane structure-containing composition | | Immediate wrinkle improving effect | ○ | ⊚ | ⊚ | ⊚ | ⊚ | ○ | ○ | △ | △ |
| | | Continuous wrinkle improving effect | ⊚ | ⊚ | ⊚ | ⊚ | ⊚ | ○ | ○ | △ | △ |
| | | Immediate elasticity improving effect | ○ | ⊚ | ⊚ | ⊚ | ⊚ | ○ | ○ | △ | △ |
| | | Continuous elasticity improving effect | ○ | ⊚ | ⊚ | ⊚ | ⊚ | ○ | ○ | △ | △ |
| | | Immediate radiance improving effect | ○ | ⊚ | ⊚ | ⊚ | ⊚ | ○ | ○ | △ | △ |
| | Skin improving effect | Continuous radiance improving effect | ⊚ | ⊚ | ⊚ | ⊚ | ⊚ | ○ | ⊚ | △ | △ |
| | | Immediate texture improving effect | ○ | ⊚ | ⊚ | ⊚ | ⊚ | ○ | ○ | △ | △ |
| | | Continuous texture improving effect | ⊚ | ⊚ | ⊚ | ⊚ | ⊚ | ○ | ⊚ | △ | △ |
| | | Immediate dullness improving effect | ○ | ⊚ | ⊚ | ⊚ | ⊚ | ○ | ○ | △ | △ |
| | | Continuous dullness improving effect | ⊚ | ⊚ | ⊚ | ⊚ | ⊚ | ○ | ○ | △ | △ |
| | | Redness improving effect | ○ | ⊚ | ⊚ | ⊚ | ⊚ | ○ | ⊚ | △ | △ |
| | | Itching improving effect | ○ | ⊚ | ⊚ | ⊚ | ⊚ | ○ | ○ | △ | △ |
| | | Rough skin (intrinsic due to menstrual cycle) preventing effect | ⊚ | ⊚ | ⊚ | ⊚ | ⊚ | ○ | ○ | △ | △ |
| | | Rough skin (extrinsic due to seasonal change, pollen, wearing a mask, etc.) preventing effect | ⊚ | ⊚ | ⊚ | ⊚ | ⊚ | ○ | ○ | △ | △ |
| | | Sagging improving effect | ○ | ⊚ | ⊚ | ⊚ | ⊚ | ○ | ○ | △ | △ |
| | | Dark circle improving effect | ○ | ⊚ | ⊚ | ⊚ | ⊚ | ○ | ○ | △ | △ |

As shown in Table 2, the compositions of Examples 8 to 14 in which lipid membrane structures formed from hydrogenated and unhydrogenated lecithins having an acid value of 5 mg KOH/g or more were dispersed were found to have skin improving effects such as wrinkle improvement and elasticity improvement. By contrast, in Comparative Example 3 using hydrogenated lecithin having an acid value of less than 5 mg KOH/g, the skin improving effects were insufficient, though the formation of a lipid membrane structure was able to be confirmed. In Comparative Example 4, albeit using hydrogenated lecithin having an acid value of 5 mg KOH/g or more, an O/W emulsion, not a lipid membrane structure, was formed due to blending of an excess of squalane, and the skin improving effects were insufficient.

## Claims

1. A pore improving agent comprising a composition containing a lipid membrane structure formed from a phospholipid having an acid value of 5 mg KOH/g or more.

2. The pore improving agent according to claim 1, wherein the phospholipid is a hydrogenated phospholipid.

3. The pore improving agent according to claim 1 or 2, wherein the lipid membrane structure is a single-layer lamellar structure.

4. The pore improving agent according to claim 1 or 2, wherein an average hydrodynamic diameter of the lipid membrane structure is 200 nm or smaller.

5. The pore improving agent according to claim 1 or 2, wherein the pore improving agent has a pore tightening effect.

6. The pore improving agent according to claim 1 or 2, wherein the pore improving agent has a sebum secretion regulating effect.

7. The pore improving agent according to claim 1 or 2, wherein the pore improving agent has an effect of surfacing or shedding keratin plugs or an effect of suppressing keratin plug formation, or both.

8. The pore improving agent according to claim 1 or 2, wherein the pore improving agent has an effect of soothing acnes or an effect of suppressing acne development, or both.

9. The pore improving agent according to claim 1 or 2, wherein the pore improving agent has a pore blackhead improving effect.

10. The pore improving agent according to claim 1 or 2, wherein the pore improving agent has an effect of improving makeup adherence or an effect of improving makeup durability, or both.

11. A skin improving agent comprising a composition containing a lipid membrane structure formed from a phospholipid having an acid value of 5 mg KOH/g or more.

12. The skin improving agent according to claim 11, wherein the phospholipid is a hydrogenated phospholipid.

13. The skin improving agent according to claim 11 or 12, wherein the lipid membrane structure is a single-layer lamellar structure.

14. The skin improving agent according to claim 11 or 12, wherein an average hydrodynamic diameter of the lipid membrane structure is 200 nm or smaller.

15. The skin improving agent according to claim 11 or 12, wherein the skin improving agent has a wrinkle improving effect.

16. The skin improving agent according to claim 11 or 12, wherein the skin improving agent has an elasticity improving effect.

17. The skin improving agent according to claim 11 or 12, wherein the skin improving agent has a radiance improving effect.

18. The skin improving agent according to claim 11 or 12, wherein the skin improving agent has a texture improving effect.

19. The skin improving agent according to claim 11 or 12, wherein the skin improving agent has a dullness improving effect.

20. The skin improving agent according to claim 11 or 12, wherein the skin improving agent has a redness improving effect.

21. The skin improving agent according to claim 11 or 12, wherein the skin improving agent has an itching improving effect.

22. The skin improving agent according to claim 11 or 12, wherein the skin improving agent has a rough skin preventing effect.

23. The skin improving agent according to claim 11 or 12, wherein the skin improving agent has a sagging improving effect.

24. The skin improving agent according to claim 11 or 12, wherein the skin improving agent has a dark circle improving effect.
